# EUROPEAN PATENT SPECIFICATION

(11) **EP 4 324 408 B1**
(45) Date of publication and mention of the grant of the patent: **17.12.2025**
(21) Application number: 23759066.6
(22) Date of filing: 15.02.2023
(51) Int. Cl.: A61B 17/06

(54) **MEDICAL SUTURE YARN AND MEDICAL SUTURE DEVICE**
MEDIZINISCHES NAHTGARN UND MEDIZINISCHE NAHTVORRICHTUNG
FIL DE SUTURE MÉDICALE ET DISPOSITIF DE SUTURE MÉDICALE

(30) Priority: 25.02.2022 CN 202210177012; 25.02.2022 CN 202220404808 U
(43) Date of publication of application: 21.02.2024
(73) Proprietor: Genesis Medtech Surgical Device (Wuxi) Co., Ltd, Wuxi, Jiangsu 214062 (CN)
(72) Inventor: GE, Naijun, Wuxi, Jiangsu 214062 (CN); CHENG, Luda, Wuxi, Jiangsu 214062 (CN)
(74) Representative: Hecht, Jan-David
(86) International application number: PCT/CN2023/076127
(87) International publication number: WO 2023/160441

(56) References cited:
- EP-A1- 3 721 813
- CN-A- 113 631 102
- CN-U- 201 691 979
- CN-U- 217 592 952
- JP-A- H1 085 225
- US-A1- 2007 257 395
- US-A1- 2021 259 686
- US-A1- 2021 386 423
- US-A1- 2021 386 423

## Description

### TECHNICAL FIELD

The present invention relates to the technical field of medical instruments, and more particularly, to a medical suture and a medical suture device.

### BACKGROUND

A surgical suture is used for seaming wounds and operative incisions, and used for repairing damaged or cut muscles, blood vessels and tissues. An existing surgical suture comprises a suture with barbs, which is designed to be provided with protruding barbs on a suture body. The barbs are designed to be convenient to penetrate through tissues when pulled in a first direction, and to keep the tension of tissues when pulled in an opposite second direction. These barbs enable the suture to be used for seaming wounds, closing tissues, tensioning tissues and attaching a repair device without knotting. The suture with the barbs may be properly tensioned and fixed by applying tension to the suture.

In an existing suture with barbs, the barbs generally have the problems of insufficient structural strength, insufficient tissue fitting, and large tissue trauma caused by large barbs. On this basis, the present application is put forward.

EP 3 721 813 A1 shows a wound closure device having an elongated body with projections protruding from the elongated body wherein projections are in the shape of fin-like angular anchoring elements.

US 2021 0 259 686 A1 shows a medical suture according to the preamble of claim 1.

### SUMMARY

The invention is defined in the appended set of claims.

A first technical problem to be solved by the present invention is that an existing medical suture causes large tissue trauma.

A second technical problem to be solved by the present invention is that the existing medical suture has a low structural strength.

A third technical problem to be solved by the present invention is that the existing medical suture and tissues are a poor fit.

Aiming at the above technical problems, the present invention provides the following technical solutions:
A medical suture comprises: a suture body, wherein the suture body defines a longitudinal center line; and a plurality of pairs of barbs arranged at intervals along a direction of the longitudinal center line, wherein each pair of barbs is symmetrically arranged on opposite sides of the suture body, the barb is provided with a first end connected with the suture body and a second end far away from the suture body, and a ratio of a transverse distance L1 of second ends of each pair of the barbs along a direction perpendicular to the longitudinal center line to a transverse dimension L0 of the suture body perpendicular to the direction of the longitudinal center line ranges from 1.5 to 2.5.

In some embodiments of the present invention, a cross section of the suture body in an area without the barbs is approximately rectangular, and the suture body comprises a first end face and a second end face which are oppositely arranged and a third end face and a fourth end face which are oppositely arranged, wherein the barbs are connected onto the first end face and the second end face, a width D1 of the first end face ranges from 0.2 mm to 0.6 mm, and a width D2 of the third end face ranges from 0.2 mm to 0.6 mm.

In some embodiments of the present invention, the cross section of the suture body in the area without the barbs is approximately square.

In some embodiments of the present invention, a cross section of the suture body in an area with the barbs is approximately spindle-shaped.

According to the present invention, the barb comprises an outside face and an inside face, the outside face extends to the second end of the barb along a first part of the suture body, the inside face extends to the second end of the barb along a second part of the suture body, and a ratio of a longitudinal dimension L2 of the barb along the direction of the longitudinal center line to the transverse dimension L0 of the suture body perpendicular to the direction of the longitudinal center line ranges from 1.8 to 2.8.

In some embodiments of the present invention, a distance L3 between the first part and the second part along the direction of the longitudinal center line ranges from 0.4 mm to 1 mm.

According to the present invention, the outside face is approximately an arc face, and the inside face is approximately a straight face.

According to the present invention, a radius r1 of the arc face of the outside face ranges from 1 mm to 2 mm.

In some embodiments of the present invention, an included angle between an extension face of the inside face and the longitudinal center line ranges from 25 degrees to 45 degrees.

In some embodiments of the present invention, the outside face and the inside face are in transition through a first arc face, and a radius r2 of the first arc face ranges from 0.02 mm to 0.05 mm.

In some embodiments of the present invention, the outside face and the suture body are in transition through a second arc face, and a radius r3 of the second arc face ranges from 0.5 mm to 1.5 mm.

In some embodiments of the present invention, the inside face and the suture body are in transition through a third arc face, and a radius r4 of the third arc face ranges from 0.02 mm to 0.05 mm.

In some embodiments of the present invention, the barbs are equidistantly distributed along an extension direction of the longitudinal center line, and a distance T between adjacent barbs ranges from 1 mm to 2.5 mm.

In some embodiments of the present invention, the barbs are unequidistantly distributed along an extension direction of the longitudinal center line, and a distance T between adjacent barbs ranges from 1 mm to 2.5 mm.

In some embodiments of the present invention, the barbs extend towards the same direction to form a unidirectional barb suture.

In some embodiments of the present invention, the suture body forms a transverse center line at a first set position, the transverse center line is perpendicular to the longitudinal center line, and the barbs on two sides of the transverse center line extend towards opposite directions.

The present invention provides a medical suture device at the same time, which comprises the medical suture according to one of the embodiments above, and further comprises a suture needle arranged at a first end of the medical suture and a tail ring arranged at a second end of the medical suture; and the medical suture comprises a barb section located at a middle part and provided with a barb structure, and guide sections located at end parts and not provided with a barb structure, and the suture needle and the medical suture, and the tail ring and the medical suture are in transition through the guide section.

The present invention provides a medical suture device at the same time, which comprises the medical suture according to one of the embodiments above, and suture needles respectively arranged at two ends of the medical suture; and the medical suture comprises a first barb section and a second barb section with a barb structure, wherein the first barb section and the second barb section are connected through a transition section, and guide sections located at end parts and not provided with a barb structure, wherein the suture needles and the medical suture are in transition through the guide sections.

Compared with the prior art, the technical solutions of the present invention have the following technical effects:
In the medical suture and the medical suture device provided by the present invention, compared with an existing suture with the same specification, a transverse distance between tail ends of each pair of barbs is small, and a contact area with tissues along the direction of the longitudinal center line of the suture is reduced by nearly 50%, which is more beneficial for the barb threads to penetrate into the tissues, and beneficial to unidirectionally slide in the tissues, and meanwhile, a cut area of the tissues can be reduced, thus reducing tissue trauma.

### BRIEF DESCRIPTION OF THE DRAWINGS

The preferred embodiments in the present invention will be described in detail hereinafter with reference to the drawings, which will be helpful to understand the objects and advantages of the present invention, wherein:
FIG. 1 is a stereoscopic diagram of a specific embodiment of a medical suture provided by the present invention;
FIG. 2 is a schematic structural diagram of the specific embodiment of the medical suture provided by the present invention;
FIG. 3 is a schematic structural diagram of a medical suture device of a unidirectional equidistant medical suture provided by the present invention;
FIG. 4 is a schematic structural diagram of a medical suture device of a bidirectional equidistant medical suture provided by the present invention;
FIG. 5 is a schematic structural diagram of a medical suture device of a unidirectional unequidistant medical suture provided by the present invention; and
FIG. 6 is a schematic structural diagram of a medical suture device of a bidirectional unequidistant medical suture provided by the present invention.

### DETAILED DESCRIPTION

The technical solutions of the present invention are clearly and completely described hereinafter with reference to the drawings. Obviously, the described embodiments are only some but not all of the embodiments of the present invention. Based on the embodiments in the present invention, all other embodiments obtained by those of ordinary skills in the art without going through any creative work should fall within the scope of protection of the present invention.

In the description of the present invention, it should be noted that the orientation or position relationship indicated by the terms "center", "up", "down", "left", "right", "vertical", "horizontal", "inside", "outside", and the like is based on the orientation or position relationship shown in the drawings, it is only for the convenience of description of the present invention and simplification of the description, and it is not to indicate or imply that the indicated device or element must have a specific orientation, and be constructed and operated in a specific orientation. Therefore, the terms should not be understood as limiting the present invention. Moreover, the terms "first", "second" and "third" are used for descriptive purposes only and cannot be understood as indicating or implying relative importance.

In the description of the present invention, it should be noted that the terms "installation", "connected" and "connection" should be understood in a broad sense unless otherwise specified and defined explicitly. For example, they may be fixed connection, removable connection or integrated connection; and may be direct connection, or indirect connection through an intermediate medium, and connection inside two elements. The specific meanings of the above terms in the present invention can be understood in a specific case by those of ordinary skills in the art.

In addition, the technical features involved in different embodiments of the present invention described hereinafter may be combined with each other as long as they do not conflict with each other.

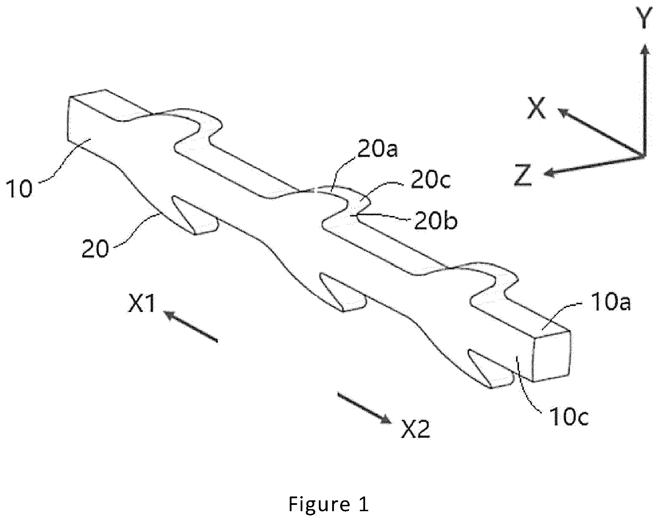

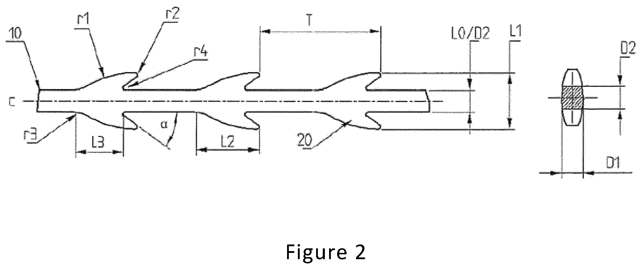

FIG. 1 and FIG. 2 show a specific embodiment of a medical suture provided by the present invention, and the medical suture is used for seaming wounds, closing tissues, tensioning tissues and attaching a repair device. The suture comprises a suture body 10 and a plurality of pairs of barbs 20 integrally formed on the suture body 10, wherein the barb 20 is designed to be convenient to penetrate through tissues when pulled along a first direction X1, and to fit the tissues when pulled along an opposite second direction X2, thus tensioning the tissues.

The suture body 10 defines a longitudinal center line c, and the barbs 20 are arranged at intervals along the longitudinal center line c. Each pair of barbs 20 is symmetrically arranged on opposite sides of the suture body 10, the barb 20 is provided with a first end (which may also be called a connecting end) connected with the suture body 10 and a second end (which may also be called a tail end or a free end) far away from the suture body 10, and a ratio L1/LO of a transverse distance L1 of the second ends of each pair of the barbs 20 along a direction perpendicular to the longitudinal center line c to a transverse dimension L0 of the suture body 10 perpendicular to the direction of the longitudinal center line c ranges from 1.5 to 2.5.

Compared with the existing suture with the same specification (based on a cross-sectional dimension of the suture body 10), a transverse distance between tail ends of each pair of barbs of the suture above is small, and a contact area of the barb with tissues along an X direction (which is namely the direction of the longitudinal center line c) is reduced by nearly 50%, which is more beneficial for the barb thread to penetrate into the tissues, and beneficial to unidirectionally slide in the tissues, and meanwhile, a cut area of the tissues can be reduced, thus reducing tissue trauma.

In an optional embodiment, a cross section of the suture body 10 in an area without the barbs 20 is approximately rectangular, and the suture body 10 comprises a first end face 10a and a second end face which are oppositely arranged and a third end face 10c and a fourth end face which are oppositely arranged, wherein a plane formed by an X direction and a Z direction is parallel to the first end face 10a and the second end face, and a plane formed by the X direction and a Y direction is parallel to the third end face 10c and the fourth end face. The barbs 20 are connected onto the first end face 10a and the second end face, a width D1 of the first end face 10a ranges from 0.2 mm to 0.6 mm, and a width D2 (which is namely a transverse dimension L0 of the suture body 10 perpendicular to the direction of the longitudinal center line c) of the third end face 10c ranges from 0.2 mm to 0.6 mm.

Preferably, the first end face 10a and the third end face 10c have an equal width, so that a cross section of the suture body 10 in an area without the barbs 20 is approximately square, thus making the suture body of the suture have a good bending moment consistency around Y and Z axes.

A cross section of the suture body 10 in an area with the barbs 20 is approximately spindle-shaped, which means that a cross section of the barb is gradually reduced from the suture body 10 to the tail end, which is beneficial for the barb to penetrate into tissues, and further reducing a cut area of the tissues, thus reducing tissue trauma.

Specifically, the barb 20 comprises an outside face 20a located on an outer side and an inside face 20b located on an inner side. During tissue suturing with the suture, the outside face 20a penetrates through a first side of the tissues to a second side of the tissues along with the suture body 10, while the inside face 20b abuts on the second side of the tissues after the whole barb 20 penetrates through the tissues. The outside face 20a extends to the second end of the barb 20 along a first part of the suture body 10, the inside face 20b extends to the second end of the barb 20 along a second part of the suture body 10, and a ratio of a longitudinal dimension L2 of the barb 20 along the direction of the longitudinal center line c to the transverse dimension L0 of the suture body 10 perpendicular to the direction of the longitudinal center line c ranges from 1.8 to 2.8. When the suture body is pulled in hard tissues (such as fascia and joint capsule) in a direction reversing X, an existing common barb structure is prone to elastic deformation due to a long arm of force, thus affecting a fixing effect of the tissues. However, the barb 20 in the embodiment has small transverse and longitudinal dimensions, a high structural strength, and a better grasping effect on the tissues. Meanwhile, since the barb 20 has the small transverse and longitudinal dimensions, a bending moment of the suture body of the suture around Y and Z axes is small, so that a bending radius of the suture body around the Y and Z axes is smaller, and the suture is easier to bend compared with an existing suture. Therefore, the suture body has a good compliance in both Y and Z directions, and the suture may better fit the tissues in vivo.

More specifically, a longitudinal dimension L2 of the barb 20 along the direction of the longitudinal center line c, which is namely a distance from the first part of the suture body 10 to the second end of the barb 20 along the direction of the longitudinal center line c, ranges from 0.6 mm to 1.2 mm.

A distance L3 between the first part and the second part along the direction of the longitudinal center line c ranges from 0.4 mm to 1 mm. Compared with the suture body of the same specification, each barb 20 of the suture has a higher base structure strength, and under the same stress condition, the barb 20 is less likely to deform than the barb 20 of the existing suture.

In an optional embodiment, the outside face 20a is approximately an arc face, and the inside face 20b is approximately a straight face. By setting the outside face 20a of the barb 20 as the arc face, it is convenient for the suture to penetrate into the tissues, so that the barb 20 unidirectionally slides in the tissues more smoothly; and by setting the inside face 20b of the barb 20 as the straight face structure, the barb 20 fits the tissues more closely, so that a grasping force to the tissues is greater and more stable. More specifically, a radius r1 of the arc face of the outside face ranges from 1 mm to 2 mm. An included angle α between an extension face of the inside face 20b and the longitudinal center line c ranges from 25 degrees to 45 degrees.

In an optional embodiment, the outside face 20a and the inside face 20b are in transition through a first arc face 20c, and a radius r2 of the first arc face 20c ranges from 0.02 mm to 0.05 mm. Compared with the structure of the barb 20 of the existing suture, the first arc face 20c at the tail end of the barb 20 in the embodiment is small, and the barb is easier to penetrate into the tissues through a design of similar sharp angle, thus being beneficial for the suture body to fix the tissues in vivo.

In an optional embodiment, the outside face 20a and the suture body 10 are in transition through a second arc face, and a radius r3 of the second arc face ranges from 0.5 mm to 1.5 mm. The inside face 20b and the suture body 10 are in transition through a third arc face, and a radius r4 of the third arc face ranges from 0.02 mm to 0.05 mm. Therefore, the barb 20 is matched with the tissues more smoothly after initially penetrating into the tissues and penetrating out of the tissues, thus reducing tissue trauma.

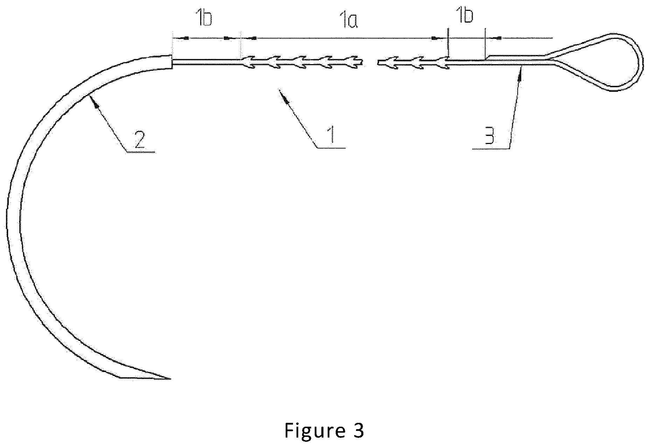

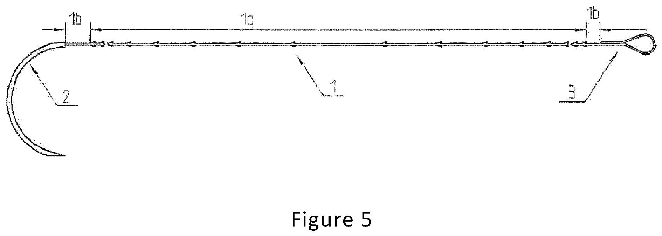

With reference to FIG. 3 and FIG. 5, the present invention provides a specific embodiment of a medical suture device, and the medical suture device comprises a medical suture 1, a suture needle 2 arranged at a first end of the medical suture 1, and a tail ring 3 arranged at a second end of the medical suture 1. Each of the barbs 20 of the medical suture 1 arranged at intervals along the longitudinal center line c extends in the same direction to form a unidirectional barb suture 1. The medical suture 1 comprises a barb section 1a located at a middle part and provided with a barb structure, and guide sections 1b located at end parts and not provided with a barb structure, and the suture needle 2 and the medical suture 1, and the tail ring 3 and the medical suture 1 are in transition through the guide section 1b.

In an optional embodiment, with reference to FIG. 3, the barbs 20 are equidistantly distributed along an extension direction of the longitudinal center line c, and a distance T between adjacent barbs 20 ranges from 1 mm to 2.5 mm. In another optional embodiment, with reference to FIG. 5, according to a specific tissue to be sutured, the barbs 20 are unequidistantly distributed along the extension direction of the longitudinal center line c, and the distance T between the adjacent barbs 20 ranges from 1 mm to 2.5 mm, so that the barbs 20 have a better adaptability with the specific tissue to be sutured.

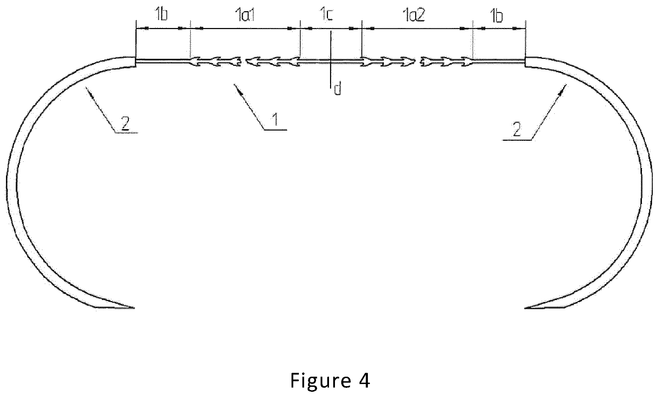

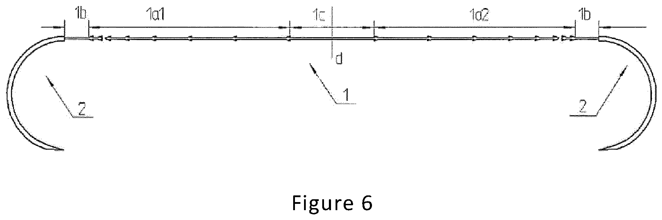

With reference to FIG. 4 and FIG. 6, the present invention provides another specific embodiment of a medical suture device, and the medical suture device comprises a medical suture 1 and suture needles 2 respectively arranged at two ends of the medical suture 1. The suture body 10 of the medical suture 1 forms a transverse center line at a first set position d, the transverse center line d is perpendicular to the longitudinal center line c, and the barbs 20 located on two sides of the transverse center line d extend in opposite directions to form a bidirectional barb suture 1. The medical suture 1 comprises a first barb section 1a1 and a second barb section 1a2 with a barb structure, wherein the first barb section 1a1 and the second barb section 1a2 are connected through a transition section 1c, and guide sections 1b located at end parts and not provided with a barb structure, wherein the barbs of the first barb section 1a1 and the second barb section 1a2 are oppositely arranged, and the suture needles 2 and the medical suture 1 are in transition through the guide sections 1b.

In an optional embodiment, with reference to FIG. 4, the barbs 20 are equidistantly distributed along an extension direction of the longitudinal center line c, and a distance T between adjacent barbs 20 ranges from 1 mm to 2.5 mm. In another optional embodiment, with reference to FIG. 6, according to a specific tissue to be sutured, the barbs 20 are unequidistantly distributed along the extension direction of the longitudinal center line c, and the distance T between the adjacent barbs 20 ranges from 1 mm to 2.5 mm, so that the barbs 20 have a better adaptability with the specific tissue to be sutured.

Obviously, the above embodiments are only examples for the purpose of clear illustration, but are not intended to limit the embodiments. For those of ordinary skills in the art, other different forms of changes or variations may be made on the basis of the above illustration. It is not necessary or possible to exhaust all the embodiments here. Moreover, the obvious changes or variations derived from this are still included within the scope of protection of the present invention, which scope is defined by the independent claim.

## Claims

1. A medical suture (1), comprising:
a suture body (10), wherein the suture body (10) defines a longitudinal center line (c); and
a plurality of pairs of barbs (20) arranged at intervals along a direction of the longitudinal center line (c), wherein each pair of barbs (20) is symmetrically arranged on opposite sides of the suture body (10), the barb (20) is provided with a first end connected with the suture body (10) and a second end far away from the suture body (10), and a ratio of a transverse distance L1 of second ends of each pair of the barbs (20) along a direction perpendicular to the longitudinal center line (c) to a transverse dimension L0 of the suture body (10) perpendicular to the direction of the longitudinal center line (c) ranges from 1.5 to 2.5,
wherein the barb (20) comprises an outside face (20a) and an inside face (20b), the outside face (20a) extends to the second end of the barb (20) along a first part of the suture body (10), the inside face (20b) extends to the second end of the barb (20) along a second part of the suture body (10), and a ratio of a longitudinal dimension L2 of the barb (20) along the direction of the longitudinal center line (c) to the transverse dimension L0 of the suture body (10) perpendicular to the direction of the longitudinal center line (c) ranges from 1.8 to 2.8,
**characterized in that** the outside face (20a) is approximately an arc face, and the inside face (20b) is approximately a straight face,
wherein a radius r1 of the arc face of the outside face (20a) ranges from 1 mm to 2 mm.

2. The medical suture (1) according to claim 1, **characterized in that** a cross section of the suture body (10) in an area without the barbs (20) is approximately rectangular, and the suture body (10) comprises a first end face (10a) and a second end face which are oppositely arranged and a third end face (10c) and a fourth end face which are oppositely arranged, wherein the barbs (20) are connected onto the first end face (10a) and the second end face, a width D1 of the first end face (10a) ranges from 0.2 mm to 0.6 mm, and a width D2 of the third end face (10c) ranges from 0.2 mm to 0.6 mm, preferably, the cross section of the suture body (10) in the area without the barbs (20) is approximately square.

3. The medical suture (1) according to claim 2, **characterized in that** a cross section of the suture body (10) in an area with the barbs (20) is approximately spindle-shaped.

4. The medical suture (1) according to claim 1, **characterized in that** a distance L3 between the first part and the second part along the direction of the longitudinal center line (c) ranges from 0.4 mm to 1 mm.

5. The medical suture (1) according to any of claims 1 to 4, **characterized in that** an included angle between an extension face of the inside face (20b) and the longitudinal center line (c) ranges from 25 degrees to 45 degrees.

6. The medical suture (1) according to any of claims 1 to 5, **characterized in that** the outside face (20a) and the inside face (20b) are in transition through a first arc face, and a radius r2 of the first arc face ranges from 0.02 mm to 0.05 mm.

7. The medical suture (1) according to any of claims 1 to 6, **characterized in that** the outside face (20a) and the suture body (10) are in transition through a second arc face, and a radius r3 of the second arc face ranges from 0.5 mm to 1.5 mm.

8. The medical suture (1) according to any of claims 1 to 7, **characterized in that** the inside face (20b) and the suture body (10) are in transition through a third arc face, and a radius r4 of the third arc face ranges from 0.02 mm to 0.05 mm.

9. The medical suture (1) according to claim 1, **characterized in that** the barbs (20) are equidistantly or unequidistantly distributed along an extension direction of the longitudinal center line (c), and a distance T between adjacent barbs (20) ranges from 1 mm to 2.5 mm.

10. The medical suture (1) according to claim 1, **characterized in that** the barbs (20) extend towards the same direction to form a unidirectional barb suture.

11. The medical suture (1) according to claim 1, **characterized in that** the suture body (10) forms a transverse center line (d) at a first set position, the transverse center line (d) is perpendicular to the longitudinal center line (c), and the barbs (20) on two sides of the transverse center line (d) extend towards opposite directions.

12. A medical suture device, comprising the medical suture (1) according to any one of claims 1 to 10, and further comprising a suture needle (2) arranged at a first end of the medical suture (1) and a tail ring (3) arranged at a second end of the medical suture (1);
the medical suture (1) comprising a barb section (1a) located at a middle part and provided with a barb structure, and guide sections (1b) located at end parts and not provided with a barb structure, and the suture needle (2) and the medical suture (1), and the tail ring (3) and the medical suture (1) being in transition through the guide section (1b).

13. A medical suture device, comprising the medical suture (1) according to claim 11,
and suture needles (2) respectively arranged at two ends of the medical suture (1);
the medical suture (1) comprising a first barb section (1a1) and a second barb section (1a2) with a barb structure, the first barb section (1a1) and the second barb section (1a2) being connected through a transition section (1c); and guide sections (1b) located at end parts and not provided with a barb structure, the suture needles (2) and the medical suture (1) being in transition through the guide sections (1b).

## Patentansprüche

1. Medizinisches Nahtmaterial (1), umfassend:
ein Nahtmaterialkörper (10), wobei der Nahtmaterialkörper (10) eine Längsmittellinie (c) definiert; und eine Vielzahl von Paaren von Widerhaken (20), die in Abständen entlang einer Richtung der Längsmittellinie (c) angeordnet sind, wobei jedes Paar von Widerhaken (20) auf gegenüberliegenden Seiten des Nahtmaterialkörpers (10) symmetrisch angeordnet ist, der Widerhaken (20) mit einem ersten Ende bereitgestellt ist, das mit dem Nahtmaterialkörper (10) verbunden ist, und einem zweiten Ende bereitgestellt ist, das weit von dem Nahtmaterialkörper (10) entfernt ist, und ein Verhältnis eines Querabstands L1 der zweiten Enden jedes Paares von Widerhaken (20) entlang einer Richtung senkrecht zur Längsmittellinie (c) zu einer Querabmessung L0 des Nahtmaterialkörpers (10) senkrecht zur Richtung der Längsmittellinie (c) im Bereich von 1,5 bis 2,5 liegt,
wobei der Widerhaken (20) eine Außenfläche (20a) und eine Innenfläche (20b) umfasst, die Außenfläche (20a) sich bis zum zweiten Ende des Widerhakens (20) entlang eines ersten Teils des Nahtmaterialkörpers (10) erstreckt, die Innenfläche (20b) sich bis zum zweiten Ende des Widerhakens (20) entlang eines zweiten Teils des Nahtmaterialkörpers (10) erstreckt, und ein Verhältnis einer Längsabmessung L2 des Widerhakens (20) entlang der Richtung der Längsmittellinie (c) zu der Querabmessung L0 des Nahtmaterialkörpers (10) senkrecht zur Richtung der Längsmittellinie (c) im Bereich von 1.8 bis 2,8 liegt,
**dadurch gekennzeichnet, dass** die Außenfläche (20a) annähernd eine Bogenfläche und die Innenfläche (20b) annähernd eine gerade Fläche ist,
wobei ein Radius r1 der Bogenfläche der Außenfläche (20a) im Bereich von 1 mm bis 2 mm liegt.

2. Medizinisches Nahtmaterial (1) nach Anspruch 1, **dadurch gekennzeichnet, dass** ein Querschnitt des Nahtmaterialkörpers (10) in einem Bereich ohne die Widerhaken (20) annähernd rechteckig ist, und der Nahtmaterialkörper (10) eine erste Endfläche (10a) und eine zweite Endfläche umfasst, die einander gegenüberliegend angeordnet sind, und eine dritte Endfläche (10c) und eine vierte Endfläche umfasst, die einander gegenüberliegend angeordnet sind, wobei die Widerhaken (20) mit der ersten Endfläche (10a) und der zweiten Endfläche verbunden sind, eine Breite D1 der ersten Endfläche (10a) im Bereich von 0.2 mm bis 0,6 mm und eine Breite D2 der dritten Endfläche (10c) im Bereich von 0,2 mm bis 0,6 mm liegt, wobei vorzugsweise der Querschnitt des Nahtmaterialkörpers (10) in dem Bereich ohne die Widerhaken (20) annähernd quadratisch ist.

3. Medizinisches Nahtmaterial (1) nach Anspruch 2, **dadurch gekennzeichnet, dass** ein Querschnitt des Nahtmaterialkörpers (10) in einem Bereich mit den Widerhaken (20) annähernd spindelförmig ist.

4. Medizinisches Nahtmaterial (1) nach Anspruch 1, **dadurch gekennzeichnet, dass** ein Abstand L3 zwischen dem ersten Teil und dem zweiten Teil entlang der Richtung der Längsmittellinie (c) im Bereich von 0,4 mm bis 1 mm liegt.

5. Medizinisches Nahtmaterial (1) nach einem der Ansprüche 1 bis 4, **dadurch gekennzeichnet, dass** ein Öffnungswinkel zwischen einer Verlängerungsfläche der Innenfläche (20b) und der Längsmittellinie (c) im Bereich von 25 Grad und 45 Grad liegt.

6. Medizinisches Nahtmaterial (1) nach einem der Ansprüche 1 bis 5, **dadurch gekennzeichnet, dass** die Außenfläche (20a) und die Innenfläche (20b) durch eine erste Bogenfläche ineinander übergehen und ein Radius r2 der ersten Bogenfläche im Bereich von 0,02 mm und 0,05 mm liegt.

7. Medizinisches Nahtmaterial (1) nach einem der Ansprüche 1 bis 6, **dadurch gekennzeichnet, dass** die Außenfläche (20a) und der Nahtmaterialkörper (10) durch eine zweite Bogenfläche ineinander übergehen und ein Radius r3 der zweiten Bogenfläche im Bereich von 0,5 mm und 1,5 mm liegt.

8. Medizinisches Nahtmaterial (1) nach einem der Ansprüche 1 bis 7, **dadurch gekennzeichnet, dass** die Innenfläche (20b) und der Nahtmaterialkörper (10) durch eine dritte Bogenfläche ineinander übergehen und ein Radius r4 der dritten Bogenfläche im Bereich von 0,02 mm und 0,05 mm liegt.

9. Medizinisches Nahtmaterial (1) nach Anspruch 1, **dadurch gekennzeichnet, dass** die Widerhaken (20) entlang einer Erstreckungsrichtung der Längsmittellinie (c) gleichmäßig oder ungleichmäßig verteilt sind und ein Abstand T zwischen benachbarten Widerhaken (20) im Bereich von 1 mm bis 2,5 mm liegt.

10. Medizinisches Nahtmaterial (1) nach Anspruch 1, **dadurch gekennzeichnet, dass** sich die Widerhaken (20) in dieselbe Richtung erstrecken, um ein unidirektionales Widerhakennahtmaterial auszubilden.

11. Medizinisches Nahtmaterial (1) nach Anspruch 1, **dadurch gekennzeichnet, dass** der Nahtmaterialkörper (10) an einer ersten Sollposition eine Quermittellinie (d) ausbildet, die Quermittellinie (d) senkrecht zur Längsmittellinie (c) steht und sich die Widerhaken (20) auf zwei Seiten der Quermittellinie (d) in entgegengesetzte Richtungen erstrecken.

12. Medizinische Nahtmaterialvorrichtung, umfassend das medizinische Nahtmaterial (1) nach einem der Ansprüche 1 bis 10 und ferner umfassend eine Nahtmaterialnadel (2), die an einem ersten Ende des medizinischen Nahtmaterials (1) angeordnet ist, und einen Abschlußring (3), der an einem zweiten Ende des medizinischen Nahtmaterials (1) angeordnet ist; wobei das medizinische Nahtmaterial (1) einen Widerhakenabschnitt (1a) umfasst, der sich an einem mittleren Teil befindet und mit einer Widerhakenstruktur bereitgestellt ist, sowie Führungsabschnitte (1b), die sich an Endteilen befinden und nicht mit einer Widerhakenstruktur bereitgestellt sind, und wobei die Nahtmaterialnadel (2) und das medizinische Nahtmaterial (1) sowie der Abschlußring (3) und das medizinische Nahtmaterial (1) durch den Führungsabschnitt (1b) ineinander übergehen.

13. Medizinische Nahtmaterialvorrichtung, umfassend das medizinische Nahtmaterial (1) nach Anspruch 11 und Nahtmaterialnadeln (2), die jeweils an zwei Enden des medizinischen Nahtmaterials (1) angeordnet sind;
die medizinische Nahtmaterialvorrichtung (1), umfassend einen ersten Widerhakenabschnitt (1a1) und einen zweiten Widerhakenabschnitt (1a2) mit einer Widerhakenstruktur, wobei der erste Widerhakenabschnitt (1a1) und der zweite Widerhakenabschnitt (1a2) durch einen Übergangsabschnitt (1c) verbunden sind;
und Führungsabschnitte (1b), die sich an den Endteilen befinden und nicht mit einer Widerhakenstruktur bereitgestellt sind, wobei die Nahtmaterialnadeln (2) und das medizinische Nahtmaterial (1) durch die Führungsabschnitte (1b) ineinander übergehen.

## Revendications

1. Suture médicale (1), comprenant :
un corps de suture (10), dans lequel le corps de suture (10) définit une ligne centrale longitudinale (c) ; et
une pluralité de paires de barbes (20) disposées à intervalles le long d'une direction de la ligne centrale longitudinale (c), dans laquelle chaque paire de barbes (20) est disposée symétriquement sur des côtés opposés du corps de suture (10), la barbe (20) est pourvue d'une première extrémité reliée au corps de suture (10) et d'une seconde extrémité éloignée du corps de suture (10), et un rapport d'une distance transversale L1 des secondes extrémités de chaque paire de barbes (20) le long d'une direction perpendiculairement à la ligne centrale longitudinale (c) sur une dimension transversale L0 du corps de suture (10) perpendiculairement à la direction de la ligne centrale longitudinale (c) va de 1,5 à 2,5,
dans laquelle la barbe (20) comprend une face extérieure (20a) et une face intérieure (20b), la face extérieure (20a) s'étend vers la seconde extrémité de la barbe (20) le long d'une première partie du corps de suture (10), la face intérieure (20b) s'étend vers la seconde extrémité de la barbe (20) le long d'une deuxième partie du corps de suture (10), et un rapport d'une dimension longitudinale L2 de la barbe (20) le long de la direction de la ligne centrale longitudinale (c) sur la dimension transversale L0 du corps de suture (10) perpendiculairement à la direction de la ligne centrale longitudinale (c) va de 1,8 à 2,8,
**caractérisée en ce que** la face extérieure (20a) est approximativement une face en arc, et la face intérieure (20b) est approximativement une face droite,
dans laquelle un rayon r1 de la face en arc de la face extérieure (20a) va de 1 mm à 2 mm.

2. Suture médicale (1) selon la revendication 1, **caractérisée en ce qu'**une section transversale du corps de suture (10) dans une zone sans les barbes (20) est approximativement rectangulaire, et le corps de suture (10) comprend une première face d'extrémité (10a) et une deuxième face d'extrémité qui sont disposées de manière opposée et une troisième face d'extrémité (10c) et une quatrième face d'extrémité qui sont disposées de manière opposée, dans laquelle les barbes (20) sont reliées sur la première face d'extrémité (10a) et la deuxième face d'extrémité, une largeur D1 de la première face d'extrémité (10a) va de 0,2 mm à 0,6 mm, et une largeur D2 de la troisième face d'extrémité (10c) va de 0,2 mm à 0,6 mm, de préférence, la section transversale du corps de suture (10) dans la zone sans les barbes (20) est approximativement carrée.

3. Suture médicale (1) selon la revendication 2, **dans laquelle** une section transversale du corps de suture (10) dans une zone avec les barbes (20) est approximativement en forme de broche.

4. Suture médicale (1) selon la revendication 1, **dans laquelle** une distance L3 entre la première partie et la seconde partie le long de la direction de la ligne centrale longitudinale (c) va de 0,4 mm à 1 mm.

5. Suture médicale (1) selon l'une quelconque des revendications 1 à 4, **dans laquelle** un angle inclus entre une face d'extension de la face intérieure (20b) et la ligne centrale longitudinale (c) va de 25 degrés à 45 degrés.

6. Suture médicale (1) selon l'une quelconque des revendications 1 à 5, **dans laquelle** la face extérieure (20a) et la face intérieure (20b) sont en transition à travers une première face en arc, et un rayon r2 de la première face en arc va de 0,02 mm à 0,05 mm.

7. Suture médicale (1) selon l'une quelconque des revendications 1 à 6,
**caractérisée en ce que** la face extérieure(20a) et le corps de suture (10) sont en transition à travers une seconde face en arc, et un rayon r3 de la seconde face en arc va de 0,5 mm à 1,5 mm.

8. Suture médicale (1) selon l'une quelconque des revendications 1 à 7,
**caractérisée en ce que** la face intérieure (20b) et le corps de suture (10) sont en transition à travers une troisième face en arc, et un rayon r4 de la troisième face en arc va de 0,02 mm à 0,05 mm.

9. Suture médicale (1) selon la revendication 1, **caractérisée en ce que** les barbes (20) sont distribuées de manière équidistante ou de manière non équidistante le long d'une direction d'extension de la ligne centrale longitudinale (c), et une distance T entre des barbes (20) adjacentes va de 1 mm à 2,5 mm.

10. Suture médicale (1) selon la revendication 1, **caractérisée en ce que** les barbes (20) s'étendent vers la même direction pour former une suture unidirectionnelle à barbes.

11. Suture médicale (1) selon la revendication 1, **caractérisée en ce que** le corps de suture (10) forme une ligne centrale transversale (d) sur une première position définie, la ligne centrale transversale (d) est perpendiculaire à la ligne centrale longitudinale (c), et les barbes (20) sur deux côtés de la ligne centrale transversale (d) s'étendent vers des directions opposées.

12. Dispositif de suture médicale, comprenant la suture médicale (1) selon l'une quelconque des revendications 1 à 10, et comprenant en outre une aiguille de suture (2) disposée sur une première extrémité de la suture médicale (1) et un anneau de queue (3) disposé sur une seconde extrémité de la suture médicale (1) ;
la suture médicale (1) comprend une section de barbe (1a) située sur une partie centrale et pourvue d'une structure de barbe, et des sections de guidage (1b) situées sur des parties d'extrémité et non pourvues d'une structure de barbe, et l'aiguille de suture (2) et la suture médicale (1), et l'anneau de queue (3) et la suture médicale (1) étant en transition à travers la section de guidage (1b).

13. Dispositif de suture médicale, comprenant la suture médicale (1) selon la revendication 11, et des aiguilles de suture (2) respectivement disposées sur deux extrémités de la suture médicale (1) ;
la suture médicale (1) comprenant une première section de barbe (1a1) et une seconde section de barbe (1a2) avec une structure de barbe, la première section de barbe (1a1) et la seconde section de barbe (1a2) étant reliées par l'intermédiaire d'une section de transition (1c) ; et des sections de guidage (1b) situées sur des parties d'extrémité et non pourvues d'une structure de barbe, les aiguilles de suture (2) et la suture médicale (1) étant en transition par l'intermédiaire des sections de guidage (1b).
